# EUROPEAN PATENT APPLICATION

(11) **EP 2 073 011 A1**
(43) Date of publication of application: **24.06.2009**
(21) Application number: 07024849.7
(22) Date of filing: 20.12.2007
(51) Int. Cl.: G01N 33/68, G01N 1/30

(54) **Means and methods of processing biological samples**

(71) Applicant: Max-Planck-Gesellschaft zur Förderung der Wissenschaften e.V., 80539 München (DE)
(72) Inventor: Lührmann, Reinhard, 35041 Marburg (DE); Kastner, Berthold, 35041 Marburg (DE); Stark, Holger, 37136 Waake (DE); Urlaub, Henning, 37083 Göttingen (DE); Pörschke, Dietmar, 37077 Göttingen (DE); Sander, Björn, 37077 Göttingen (DE); Golas, Monika, 37077 Göttingen (DE); Richter, Florian, 37073 Göttingen (DE)
(74) Representative: Vossius & Partner

(57) **Abstract**

This invention relates to a method of analyzing a biological sample, said sample comprising one or more (poly)peptides, said method comprising (i) bringing said biological sample into contact with a cross-linking fixative; (ii) neutralizing any excess of said cross-linking fixative; (iii) adsorbing the sample fixated in (i) on the surface of a solid support; (iv) adding one or more endoproteases; and (v) detecting of one or more cleavage products of said endoprotease(s) by mass spectrometry. Also provided is a method of processing a biological sample, said sample comprising one or more biological macromolecules, said method comprising exposing the sample to elevated hydrostatic pressure and, either concomitantly and/or subsequently, to a cross-linking fixative. Furthermore provided are kits useful for performing the methods of the invention.

## Description

This invention relates to a method of analyzing a biological sample, said sample comprising one or more (poly)peptides, said method comprising (i) bringing said biological sample into contact with a cross-linking fixative; (ii) neutralizing any excess of said cross-linking fixative; (iii) adsorbing the sample fixated in (i) on the surface of a solid support; (iv) adding one or more endoproteases; and (v) detecting of one or more cleavage products of said endoprotease(s) by mass spectrometry.

In this specification, a number of documents including patent applications and manufacturer's manuals is cited. The disclosure of these documents, while not considered relevant for the patentability of this invention, is herewith incorporated by reference in its entirety.

The frequently very complex composition of biological samples remains a challenging task for analytical methods. The identity of certain sample constituents often remains obscure or constituents escape detection altogether. In the field of imaging methods the way samples are prepared influence the resolution of the obtained images, quite often in an uncontrolled or hardly understood manner. Resolution significantly decreases in case of structural heterogeneity, including compositional and conformational heterogeneity.

Nakaya and Wool (Biochem. Biophys. Res. Comm. 54, 239-245 (1973)) describe the association/dissociation behaviour of ribosomal subunits during centrifugation. In several experiments, glutaraldehyde is used as a fixative. Fixation is performed prior to centrifugation.

Guerrero et al. (Mol. Cell. Proteomics 5, 366-378 (2006)) describe an investigation of the protein-protein interaction network of the 26S proteasome. They apply formaldehyde in vivo for freezing stable and transient interactions. Upon purification and trypsin digestion, the composition is analyzed by mass spectrometry (MS). Guerrero et al. do not adsorb the sample on the surface of a solid support prior to trypsin digestion.

Hood et al. (Proteomics 6, 4106-4114 (2006)) review MS analysis of formalin-fixed paraffin-embedded (FFPE) tissue. Protein extracts obtained from the FFPE tissue blocks are digested with trypsin in solution and subsequently analyzed by MS.

Landau and Thibodeau (Exp. Cell Res. 27, 591-594 (1962)) treated *Amoeba proteus* in a pressure cell with potassium permanganate in order to study the micromorphology of *Amoeba proteus* during pressure-induced changes. Potassium permanganate oxidizes amines present on biological macromolecules and is unsuitable for cross-linking and stabilizing of biological macromolecules and complexes thereof; see, for example, M.A. Hayat (Chapter 5 Permanganates, pages 183 - 193, in "Fixation for electron microscopy" ed. M.A. Hayat, Academic press 1981, Chapter 5 Permanganates).

In view of the state of the art, the technical problem underlying the present invention was the provision of improved or alternative means and methods of biological sample preparation for subsequent analysis.

Accordingly, this invention relates to a method of analyzing a biological sample, said sample comprising one or more (poly)peptides, said method comprising (i) bringing said biological sample into contact with a cross-linking fixative; (ii) neutralizing any excess of said cross-linking fixative; (iii) adsorbing the sample fixated in (i) on the surface of a solid support; (iv) adding one or more endoproteases; and (v) detecting of one or more cleavage products of said endoprotease(s) by mass spectrometry.

The term "analyzing" has the meaning as established in the art. Accordingly, "analyzing a sample" refers to the obtaining of information about the constituents of the sample. Such information may be any information including identity, composition, and, referring to individual molecules or aggregates or complexes thereof, shape.

The term "biological sample" refers to samples originating from or obtained from biological material. Biological materials include living species of eubacteria, eukaryotes and archaea as well as viruses. Further comprised is any material obtained therefrom. Biological samples according to the invention are those which comprise one or more species of biological macromolecules. Biological macromolecules include polymeric molecules formed by a plurality of identical or similar building blocks. Upon formation of the polymer from the monomeric building blocks, water or other small molecules may be formed as by-products. Accordingly, polycondensates are to be subsumed under the term "polymer" according to the invention. Exemplary or preferred biological macromolecules include peptides and (poly)peptides, nucleic acids, lipids and saccharides. According to the main embodiment, the biological sample comprises one or more (poly)peptides. Biological samples according to the invention may furthermore comprise small molecules which, together with macromolecules, may be subjected to analysis.

The term "(poly)peptide" embraces polypeptides and peptides. The term "polypeptide" as used herein describes a group of molecules which consist of more than 30 amino acids. In accordance with the invention, the group of polypeptides comprises "proteins" as long as the proteins consist of a single polypeptide. Also in line with the definition, the term "polypeptide" describes fragments of proteins as long as these fragments consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The term "peptide" on the other hand relates to a group of molecules which consists of less than or equal to 30 amino acids.

The term "cross-linking fixative", when used in conjunction with biological macromolecules, has an established meaning in the art. It refers to agents capable of cross-linking of two or more moieties within one macromolecule as well as of two or more moieties located on two or more distinct molecules. Cross-linking with a cross-linking fixative may rigidify flexible parts of a macromolecule and/or may freeze transient or weak intermolecular interactions. Typically, cross-linking with a cross-linking fixative involves the formation of covalent bonds between functional groups of the fixative and functional groups of the macromolecule. Suitable functional groups are known in the art and further detailed below. The recited "bringing into contact" is effected under conditions which allow the above described cross-linking to occur. Suitable conditions are conditions where the cross-linking fixative is provided in solution, for example buffered solution, and brought into contact with the molecules to be cross-linked, said molecules being, for example, in solution as well or on the surface of a support exposed to the solution comprising the cross-linking fixative.

Buffers are well known in the art and the skilled person is aware of appropriate buffers in dependency of the samples being analyzed. Common buffers comprise (pKₐ values in brackets) H₃PO₄ / NaH₂PO₄ (pK_{a,1} = 2.12), Glycine (pK_{a,1} = 2.34), Acetic acid (4.75), Citric acid (4.76), MES (6.15), Cacodylic acid (6.27), H₂CO₃ / NaHCO₃ (pK_{a,1} = 6.37), Bis-Tris (6.50), ADA (6.60), Bis-Tris Propane (pK_{a,1} = 6.80), PIPES (6.80), ACES (6.90), Imidazole (7.00), BES (7.15), MOPS (7.20), NaH₂PO₄ / Na2HP04 (pK_{a,2} = 7.21), TES (7.50), HEPES (7.55), HEPPSO (7.80), Triethanolamine (7.80), Tricine (8.10), Tris (8.10), Glycine amide (8.20), Bicine (8.35), Glycylglycine (pK_{a,2} = 8.40), TAPS (8.40), Bis-Tris Propane (pK_{a,2} = 9.00), Boric acid (H₃BO₃ / Na₂B₄O₇) (9.24), CHES (9.50), Glycine (pK_{a,2} = 9.60), NaHCO₃ / Na₂CO₃ (pK_{a,2} = 10.25), CAPS (10.40) and Na2HPO₄ / Na₃PO₄ (pK_{a,3} = 12.67). Further envisaged buffers include 20 - 50 mM HEPES-KOH (pH. 7.5 - 8.0) and 20-50 mM Triethanolamine-HCI (pH 7.0).

Furthermore, ionic strength may be adjusted, e.g., by the addition of sodium chloride and/or potassium chloride. Preferred concentrations of sodium chloride are between 0 and 2 M, preferably between 100 and 200 mM. Examples of buffers comprising sodium chloride include PBS (phosphate buffered saline) containing 1.37 M NaCl, 27 mM KCI, 43 mM Na₂HPO₄·7H₂O and 14 mM KH₂PO₄ in the 10-fold aqueous stock solution, which is adjusted to pH 7.3; SSC containing 3 M NaCl and 0.3 M sodium citrate in 20-fold aqueous stock solution, which is adjusted to pH 7.0.

The step of neutralizing any excess of said cross-linking fixative serves to prevent any cross-linking by said fixative to occur after a desired degree of cross-linking has been achieved. Neutralizing may be effected by the addition of any agent capable of reacting with those functional group(s) present on said cross-linking fixative which render(s) the cross-linking fixative capable of reacting with said (poly)peptide or said biological macromolecule. Preferably, said neutralizing agent does not interfere with endoproteinase digestion and/or electron microscopy. Agents suitable for neutralizing include agents comprising a primary amino group such as amino acids. A preferred neutralizing agent which is an amino acid is glycine although any other amino acid may be used. Another neutralizing agent according to the invention and comprising a primary amino group is Tris. Further suitable neutralizing agents include glacial acetic acid, trifluouro acetic acid (TFA), and other acids that cause pH shift. Further suitable agents are those which have a sulfhydryl group such as cysteine and β-mercaptoethanol. If the neutralizing agent is comprised in a solution, said solution may be referred to as "stop solution".

Adsorption according to the invention is a process that occurs when a liquid solute accumulates on the surface of a solid (adsorbent), forming a molecular film (the adsorbate). Adsorption according to the invention includes physisorption, i.e. adsorption in which the adsorbate adheres to the surface only through Van der Waals interactions, and chemisorption, i.e. adsorption whereby a molecule adheres to a surface through the formation of a chemical bond. Preferably, adsorption according to the invention is physisorption.

Also preferred is that the surface of the adsorbent, i.e. of the solid support, is not functionalized. Functionalization refers to a treatment of the support to introduce molecules and/or functional groups which in turn are capable of binding analytes such as the constituents of the biological sample. Accordingly, it is furthermore preferred that prior to adsorbing the solid support serving as adsorbent is free or substantially free of biological material, or that biological material, to the extent it is comprised in the adsorbent prior to adsorbing, does not or substantially not participate in the process of adsorption. In particular, it is preferred that adsorption does not involve binding of the adsorbate to specific binding molecules on the surface of the adsorbent. Accordingly, it is also preferred to use adsorbents, i.e. solid supports, which are free or substantially free of specific binding molecules. Specific binding molecules include antibodies, fragments and derivatives thereof, biotin, avidin, streptavidin, purification tags such as His-tag, GST-tag, Calmodulin, c-myc, flag, peptide epitope tags like HA and the like.

The term "endoprotease" is well known in the art and relates to enzymes capable of cleaving a peptide bond located with a polypeptide chain. "Located within" refers to those peptide bonds which are not connecting the N-terminal or the C-terminal amino acid residue to the remainder of the polypeptide chain. In other words, the products of one catalytic cycle of an endoprotease are, at least generally, two (poly)peptides as opposed to a (poly)peptide and an amino acid. Digestion of the recited (poly)peptides with one or more endoproteases yields smaller peptides and/or polypeptides which are amenable to analysis by mass spectrometry. In a preferred embodiment, digestion with one or more endoproteases is effected directly on the surface of the solid support. Alternatively, the material adsorbed in step (iii) may be removed from the surface of the solid support and subjected to digestion with one or more endoproteases in solution.

A preferred endoprotease is selected from Trypsin, Chymotrypsin, Lys-C, Glu-C/Staphylococcus V8, Asp-N, Arg-C, Proteinase K, Pepsin, Papain, Thermolysin, Elastase and Thrombin. A particularly preferred endoprotease is Trypsin.

Detecting according to step (v) of the main embodiment is effected by mass spectrometry. Mass spectrometry of peptides, more specifically of peptides obtained by digesting proteinaceous material is well established in the art. A preferred mode of ionizing is matrix-assisted laser desorption ionisation (MALDI). Alternative ionisation methods include electron impact, chemical ionisation, atmospheric pressure, fast atom bombardment, thermospray, electrospray, laser desorption ionisation (LDI), desorption ionisation on silicon (DIOS), and electrospray ionisation (ESI). A preferred analyzer is a time-of-flight analyzer. Alternative mass separation methods include or are based on magnetic sector, cyclotron resonance, ion trap, quadrupole(s), and orbitraps. Analysing of the mass spectra may be used to reveal the sequence of the peptides comprised in the digest. The identity of the (poly)peptides giving rise to the peptides may be obtained by procedures known in the art, more specifically by comparing of the sequences inferred from the mass spectra with a database of calculated peptides obtained by an in silico digestion of known proteins by the respective one or more endoproteases; see, for example Henzel et al. (Proc. Natl. Acad. Sci. USA 90, 5011-5015 (1993)). Suitable software packages comprising a database of fragments obtainable by endoproteolytic digestion of known proteins and a sequence comparison algorithm are known in the art and include search engine such as Mascot (Perkins DN, Pappin DJ, Creasy DM, Cottrell JS. Electrophoresis, 20(18) 3551-67 (1999)), Proteinpilot (Applied Biosystems, http://www3.appliedbiosystems.com/cms/groups/psm_marketing/documents/general documents/cms_042621.pdf), Sequest (Ducret A, Van Oostveen I, Eng JK, Yates JR 3rd, Aebersold R. Protein Sci. 1998 Mar;7(3):706-19), and the like.

Prior to subjecting the endoprotease digest to MS, purification may be performed. Preferred purification methods include one- or more-dimensional gel electrophoresis and/or one- or more-dimensional liquid chromatography. Alternatively, both endoprotease digestion and mass spectrometry are carried out *in situ,* i.e. directly on the surface of the solid support. In this case, MALDI is preferred.

Steps (i) to (v) of the main embodiment may be performed in the temporal order (i) - (ii) - (iii) - (iv) - (v). The method of the invention is not limited, though, to such temporal order. For example, neutralizing may alternatively be performed after adsorbing.

The present inventors surprisingly found that adsorbing of the fixated sample on the surface of a solid support enhances the quality and reproducibility of the mass spectra. Furthermore, it turns out that the sequence information obtained from the mass spectra can be assigned to known protein sequences with higher levels of confidence than in the absence of step (iii). Confidence can be measured, for example, by the average peptide score (APS) as defined in C.L. Chepanoske, B.E. Richardso, M. von Rechenberg, JM Peltier, Rapid Commun Mass Spectrom 19 (1), 3363 (2005). The method of analyzing a biological sample according to the invention may therefore be used in the analysis of complex biological samples including the analysis of the entire proteome comprised in such samples. The term "proteome" is known in the art and refers to the entirety of the (poly)peptides comprised in a sample. Accordingly, the method of the main embodiment is also referred to herein as "proteomics method". A further advantage of this method is the lesser sample amount required for mass spectrometric analysis as compared to the prior art.

The present invention furthermore provides a method of processing a biological sample, said sample comprising one or more biological macromolecules, said method comprising (a) exposing the sample to elevated hydrostatic pressure and, either concomitantly and/or subsequently, to a cross-linking fixative.

The term "hydrostatic pressure" is known in the art and relates to the pressure at a given point in a fluid at rest. The term "elevated hydrostatic pressure" refers to hydrostatic pressure which is elevated as compared to the hydrostatic pressure in a liquid at normal atmospheric pressure. Normal atmospheric pressure is a pressure of *p₀* = 101325 Pa. The hydrostatic pressure *p* in a liquid at normal atmospheric pressure is given by *p* = *p₀* + *h* ρ *g*, wherein h is the height of the liquid above the given point in the fluid, p is the density of the liquid and *g* is the gravitational acceleration. In the absence of further forces, g is the standard gravity go of 9.80665 m/s² at the earth's surface. In order to establish elevated hydrostatic pressure, *p₀* may be replaced by a total external pressure *pₑₓₜ,* wherein *pₑₓₜ* > *p₀.* Preferably, *pₑₓₜ* is at least 2 *p₀,* more preferred at least 5 *p₀,* and yet more preferred at least 10 *p₀.* Alternatively or in addition, the value of g may be increased, for example by means of centrifuging (see below).

The concomitant or subsequent exposure to a cross-linking fixative, the cross-linking fixative being defined herein above and further detailed below, freezes the state of the molecules present in the sample as obtained by the action of elevated hydrostatic pressure.

The exposure to elevated hydrostatic pressure reduces the presence of non-specific interactions and accordingly the extent of non-specific aggregation in the biological sample. It turns out that the fraction of monodisperse molecules and/or complexes present in the sample increases. The present inventors surprisingly found that a sample processed by the method of the invention exhibits better quality, for example, in subsequent imaging methods. For example, in single particle electron microscopy, particles are more homogeneous and exhibit increased image contrast. Moreover, the number of good quality class averages of single particle images increases significantly (see enclosed Examples and Figures). Class averages are the arithmetic mean of a group of electron microscopic images that have been clustered by computational classification algorithms like hierarchical ascendant classification, K-means clustering, self-organizing maps or reference-based classification by alignment. These as well as further suitable classification algorithms are known in the art. Also, an increase in projection angle diversity of the images is observed (see enclosed Examples and Figures). Projection angle diversity refers to the orientational dispersion of the macromolecules on the solid support that is caused by the degree of randomness of adsorption. A further advantage is that more particles are adsorbed per time unit and the maximum number of particles that are adsorbed is higher.

Furthermore, improvements in the quality of subsequently recorded mass spectra are observed. Accordingly, it is envisaged to use the method of processing a biological sample of the invention as a preferred embodiment of step (i) of the method of analyzing a biological sample according to the main embodiment.

In a preferred embodiment, step (a) is effected by centrifuging said biological sample in a gradient of density and in the presence of a cross-linking fixative.

Centrifuging is a preferred means of establishing elevated hydrostatic pressure. In this case, the value of g in the formula above is increased to values above go. Preferably, g is at least 2 go, more preferred at least 5 go, and yet more preferred at least 10 go.

The gradient of density permits a fractionation of the sample. Prior to centrifugation, the sample is layered on top of the gradient. During centrifugation different molecules and/or particles migrate then at different velocities in the gradient. Gradient centrifugation as such is known in the art and further described in, for example, Preparative centrifugation: a practical approach. Ed. D. Rickwood, Practical approach series, 2nd edition, Oxford University Press, 1992.

This embodiment is also referred to herein as "GraFix" method, since it employs a gradient in conjunction with a cross-linking fixative.

In a more preferred embodiment, said cross-linking fixative is provided in a concentration gradient, and wherein the directions of said gradient of density and of said concentration gradient are the same.

In this case, the method of processing a biological sample according to the invention uses a double gradient. The gradient of the cross-linking fixative may be used in those cases where a particularly gentle fixation is desired. The sample, which is layered on top of the double gradient is initially exposed to low or vanishing concentrations of the cross-linking fixative and subsequently, as centrifugation proceeds, to increasing concentrations of the cross-linking fixative.

In a further preferred embodiment, said gradient of density is a gradient of an agent, said agent being selected from the group consisting of monosaccharides, oligosaccharides, polysaccharides, alcohols with two or more -OH groups, caesium salts and iodinated compounds.

As such, any agent known in the art for the preparation of a density gradient may be used in accordance with the present invention. Preferred caesium salts are CsCl and Cs₂SO₄. Preferred iodinated compounds are Metrizamide and Nycodenz. Preferred polysaccharides include Ficoll and Dextran. Preferred oligosaccharides are trehalose and sucrose. A preferred alcohol with two or more -OH groups is glycerol.

In a further preferred embodiment, said centrifuging is performed at about 50 000 to about 1 000 000 x g for about 15 min to about 25 hours.

Preferably, the method of processing a biological sample involving centrifuging furthermore comprises (b) harvesting at least one fraction of the gradient after centrifuging according to (a).

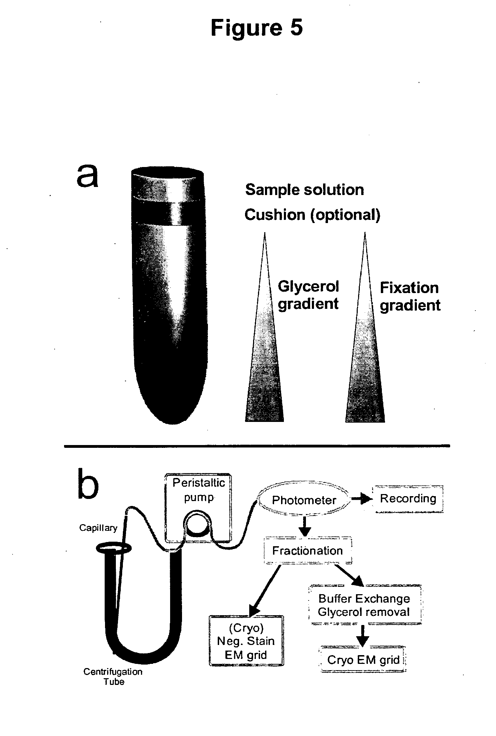

Methods of harvesting fractions after gradient centrifugation are within the skills of the person skilled in the art. Preferably, the gradient is harvested from bottom to top in order to minimize contamination of high molecular weight molecules and/or complexes with low molecular weight material from the top of the gradient (see Figure 5).

In a more preferred embodiment, (c) at least one fraction is adsorbed on the surface of a solid support. Further details and advantages of this step are discussed in conjunction with the main embodiment above. Preferably, any excess of said cross-linking fixative is neutralized prior to or after said adsorbing according to (c).

As one alternative to centrifuging, said elevated hydrostatic pressure in step (a) is created by a pressure cell. The required high pressure can be achieved by making use of a hydraulic fluid as described by Yayanos, A.A. and Pollard, E.C. (A study of the effects of hydrostatic pressure on macromolecular synthesis in Escherichia coli. Biophys J. 1969, 9:1464-82). An example for a pressure device has been described by Landau, J.V. and Thibodeau (loc. cit.).

Preferably, said elevated hydrostatic pressure is between about 5 and about 100 MPa, more preferred between 10 and 50 MPa.

In a preferred embodiment, the method of processing a biological sample further comprises, after said exposing according to (a), the steps of (b') neutralizing any excess of said cross-linking fixative; and (c') adsorbing said sample on the surface of a solid support. Optionally, step (b') may be omitted. The omission of step (b') is envisaged in particular for those cases where electron microscopy is subsequently performed, wherein said electron microscopy is not cryo-electron microscopy.

Steps (b') and (c') have been discussed in conjunction with the corresponding steps of the main embodiment herein above. It is preferred to perform step (c') after step (b'). The method is not limited in this regard, though.

In a further preferred embodiment, the method of processing a biological sample further comprises (d) adding one or more endoproteases; and (e) detecting of one or more cleavage products of said endoprotease(s) by mass spectrometry.

Steps (d) and (e) have been discussed in conjunction with the corresponding steps of the main embodiment herein above. Preferably, both endoprotease digestion and mass spectrometry are carried out *in situ,* i.e. directly on the surface of the solid support.

Preferably, digestion by said endoprotease(s) is carried out in the presence of a denaturing agent.

Any denaturing agent known in the art may be used. In particular, agents denaturing proteins and polypeptides are envisaged. Preferred denaturing agents include urea, thiourea, guanidinium hydrochloride, guanidinium thiocyanate; acetonitril, methanol, octylglycoside, sodium dodecyl sulfate (SDS) and lithium dodecyl sulfate (LDS). Preferred concentrations of the denaturing agent are between about 0.1 and about 2 M, more preferred between about 0.5 and 1.5 M, most preferred about 1 M. The presence of a denaturing agent is preferred in those cases where a maximum of information is to be obtained by subsequent analysis by mass spectrometry.

Furthermore, other agents may be added before, during or after endoprotease digestion as necessary. For example, RNases A and/or T1 and/or DNases and/or Exo- and/or Endoglycosidases may be added if hydrolyzing of RNAs or DNA present in the sample or disrupting of (e.g. posttranlational) protein modifications is desired prior to subsequent analysis by mass spectrometry (MS). The choice of these further agents depends on the nature of protein-ligand interactions to be studied as well as on presence or absence of postranslational modifications.

Alternatively, digestion of adsorbed samples without any denaturing agent is deliberately envisaged. This may be done in order to map surface and/or preferential orientation of the macromolecules. Also in case mass spectrometry is performed subsequently, no denaturing may be used.

As an alternative to endoproteolytic digestion and analysis by MS or in addition thereto, it is envisaged to perform structural analysis of the processed sample or material adsorbed on said support, respectively.

Preferably, said structural analysis is selected from electron microscopy, X-ray and light scattering and atomic force microscopy.

In a further preferred embodiment, electron microscopy (EM) is negative-stain, cryo-negative stain or unstained cryo-EM, preferably of single particles. Also, EM may be performed of thin frozen films of said fraction. In case unstained cryo-EM is envisaged, it is preferred to remove any agent used to build the density gradient from the sample prior to performing unstained cryo-EM. Such removal may be effected by using buffer exchange spin columns. In case of cryo-EM, it is preferred to neutralize any excess of said cross-linking fixative prior to performing said structural analysis.

In a preferred embodiment, said biological sample comprises a complex of biological macromolecules.

As documented in the examples enclosed herewith, the methods of the invention deliver superior performance as compared to the prior art when applied to structural analysis of macromolecular complexes such as ribosomes, spliceosomes and the anaphase-promoting complex/cyclosome (APC/C).

Further fields benefiting from the methods of the invention and from accordingly prepared biological samples are biotechnology and medicine. Preferably, the method of the invention stabilises said samples while avoiding generation of fixed aggregates. The fixed and preferably monodisperse sample as obtained by the methods of processing a biological sample according to the invention is expected to be well suited for *in vitro* (e.g. diagnostic systems, enzymatic reactions, isolation strategies) and *in vivo* (e.g. protein substitution, vaccination, targeting, drug delivery) application.

In a further preferred embodiment, said cross-linking fixative is a bi- or multifunctional reagent, wherein the functional groups of said reagent are capable of reacting with functional groups present on said biological macromolecules to form covalent bonds.

Preferred biological macromolecules include (poly)peptides as defined above.

Preferred functional groups of said reagent, i.e., of said cross-linking fixative, are Aldehyde, Aryl Acide, Carbodiimide, Hydroxymethyl Phosphine, Imidoester, Isocyanate, Diacid chloride and N-Hydroxysuccinimide-Ester (NHS). Monoaldehydes such as formaldehyde are subsumed under the term "bifunctional reagent", since monoaldehydes such as formaldehyde are capable of cross-linking two moieties of biological macromolecule by acetal formation. Preferred functional groups of the biological macromolecule, preferably of the (poly)peptide are amine, carboxyl, sulfhydryl and hydroxyl.

Preferred cross-linking fixatives are selected from the group consisting of glutaraldehyde, formaldehyde, Bis(Sulfosuccinimidyl) suberate (BS³), Dimethyl adipimidate (DMA), Dimethyl pimelimidate (DMP), Dimethyl Suberimidate (DMS), Dithiobis(succinimidyl) propionate (DSP), Disuccinimidyl suberate (DSS), β-[Tris(hydroxymethyl) phosphino] propionic acid (THPP), Carbodiimide, Galactose dialdehyde, S-Acetylthioacetic acid-N-hydroxysuccinimide ester (SATA), 3-Maleimidobenzoic acid *N*-hydroxysuccinimide ester (MBS), 1,4-Bis[3-(2-pyridyldithio)propionamido]butane (DPDPB), Bis[2-(N-succinimidyloxycarbonyloxy)ethyl] sulfone (BSOCOES), Dimethyl 3,3'-dithiopropionimidate dihydrochloride (DTBP), Sebacic acid bis(N-succinimidyl) ester (DSS), Tris-(2-carboxyethyl)phosphine hydrochloride (TCEP), 1-Ethyl-3-(3-dimethylaminopropyl) carbodiimide hydrochloride (EDAC), N-Hydroxysulfosuccinimide sodium salt (NHSS), Succinimidyl acetylthioacetate (SATA), Succinimidyl trans-4-(maleimidylmethyl) cyclohexane-1-carboxylate (SMCC), Succinimidyl 3-(2-pyridyldithio)propionate (SPDP) and mixtures thereof.

The most preferred cross-linking fixative according to the invention is glutaraldehyde. The systematic name of glutaraldehyde is pentane-1,5-dial. The use of glutaraldehyde as cross-linking fixative in the course of the preparation of samples for electron microscopy is known in the art. Properties of glutaraldehyde, commonly applied conditions for fixation with glutaraldehyde, and mechanisms of cross-linking of biomolecules by glutaraldehyde are described, for example, in the review articles of Hayat (Micron and Microscopica Acta 17, 115-135 (1986)) and Migneault et al. (BioTechniques 37, 790-802 (2004)).

In a further preferred embodiment, said cross-linking fixative is present in a concentration between about 0.001 and about 1 vol-%. More preferred are concentration between about 0.025 and about 0.1 vol-%. Most preferred is a concentration of about 0.075 vol-%.

Preferred shapes of the gradient(s) are linear and exponential, in the latter case preferably slightly exponential.

Preferred incubation times with the cross-linking fixative are between about 30 min and about 96 hours, more preferred between about 2 and about 18 hours, and yet more preferred between about 8 and about 18 hours.

In those embodiments of the method of processing a biological sample according to the invention wherein exposure to the cross-linking fixative is to be effected subsequently to the exposure to elevated hydrostatic pressure, it is envisaged that exposure to elevated hydrostatic pressure may continue during exposure to the cross-linking fixative. This may be achieved, for example, by first mixing the sample with an inactive form of the cross-linking fixative followed by application of the elevated hydrostatic pressure and final activation of the cross-linking fixative. By using reagents with Aryl Azide as a functional group activation can be achieved by UV light exposure. Possible reagents are Bis-[b-(4-Azidosalicylamido)ethyl]disulfide (BASED), N-Hydroxysuccinimidyl-4-azidosalicylic acid (NHS-ASA), 4-[p-Azidosalicylamido]butylamine (ASBA), Sulfosuccinimidyl[4-azidosalicylamido]-hexanoate (Sulfo-NHS-LC-ASA).

In a further preferred embodiment, said support is selected from carbon support, reversed phase material, nitrocellulose, PVDF, polystyrol, and silicate-based solid supports reversed phase material is known in the art and includes C18 bonded silica, C8 bonded silica and phenyl bonded silica. Generally, reversed phase material comprising alkyl chains, in particular in the range between C3 and C30, is envisaged. Preferred silicate-based supports include mica, porcelain, quartz and glass including glass beads. Preferably, said carbon support is selected from carbon films and activated carbon. Activated carbon includes activated charcoal. Particularly good performance is achieved when carbons supports are used. Carbon films to be used in accordance with the invention include carbon films commonly used in electron microscopy such as carbon-coated polyvinylformal grids. Also included are metal-doped carbon films. Suitable metals for doping include platinum.

Further solid supports which are envisaged include metals such as gold, platinum, titanium; ceramics including ceramics tempered with oxides (aluminium, magnesium zirconium and/or titanium oxide, pure or mixed forms); carbides (e.g. silicium-borate carbides); nitrides (silicon, aluminium, and/or borate nitrides); plastics including polystyrol-, polyurethane-, polysulphone-, and/or epoxy-based plastics; carbohydrate-based supports such as cellulose, dextran, sepharose, chitin; and ion exchange resins including silica-based resins, zeolites, aluminium oxide-based resins, and epoxide-based resins. As stated above, it is preferred that the surface of the solid support is not functionalized. In particular, it is preferred that the surface of the solid support is free or substantially free of biological material such as the specific binding molecules referred above.

In a further preferred embodiment, said adsorbing is allowed to occur for a period of time of at least about 5 sec. Further preferred adsorption times are at least about 20 min or between about 1.5 and 12 hours.

The present invention furthermore provides a kit comprising (a) a cross-linking fixative selected from glutaraldehyde, formaldehyde, Bis(Sulfosuccinimidyl) suberate, Dimethyl adipimidate, Dimethyl pimelimidate, Dimethyl Suberimidate, Dithiobis(succinimidyl) propionate, Disuccinimidyl suberate, β-[Tris(hydroxymethyl) phosphino] propionic acid, Carbodiimide, Galactose dialdehyde, S-Acetylthioacetic acid-N-hydroxysuccinimide ester (SATA), 3-Maleimidobenzoic acid *N-*hydroxysuccinimide ester (MBS), 1,4-Bis[3-(2-pyridyldithio)propionamido]butane (DPDPB), Bis[2-(N-succinimidyl-oxycarbonyloxy)ethyl] sulfone (BSOCOES), Dimethyl 3,3'-dithiopropionimidate dihydrochloride (DTBP), Sebacic acid bis(N-succinimidyl) ester (DSS), Tris-(2-carboxyethyl)phosphine hydrochloride (TCEP), 1-Ethyl-3-(3-dimethylaminopropyl) carbodiimide hydrochloride (EDAC), *N-*Hydroxysulfosuccinimide sodium salt (NHSS), Succinimidyl acetylthioacetate (SATA), Succinimidyl *trans*-4-(maleimidylmethyl) cyclohexane-1-carboxylate (SMCC), Succinimidyl 3-(2-pyridyldithio)propionate (SPDP) and mixtures thereof; (b) a solid support; and (c) an endoprotease. Any of the preferred cross-linking fixatives described herein above may be used as a constituent of the kit according to the invention. The kit is useful for performing the proteomics method according to the invention.

In a preferred embodiment, said support is selected from carbon support, reversed phase material, nitrocellulose, PVDF, polystyrol and silicate-based solid supports. Preferred silicate-based supports include mica.

Preferably, said kit further comprises a denaturing agent. Preferably, said denaturing agent is selected from urea, thiourea, guanidinium hydrochloride, guanidinium thiocyanate, acetonitril, methanol, octylglycoside, sodium dodecyl sulfate (SDS) and lithium dodecyl sulfate (LDS).

Preferably, said endoprotease comprised in the kit of the invention or to be used for the methods of the invention is selected from Trypsin, Chymotrypsin, Lys-C, Glu-C/Staphylococcus V8, Asp-N, Arg-C, Proteinase K, Pepsin, Papain, Thermolysin, Elastase and Thrombin. Particularly preferred is Trypsin. Preferably, said endoprotease is an endoprotease which is capable of cleaving its substrate in the presence of said denaturing agent. The skilled person is aware of such endoproteases and may select suitable endoproteases without further ado.

In a more preferred embodiment, the kit of the invention further comprises a pressure cell. Pressure cells are known in the art and described above.

In a further preferred embodiment, the kit of the invention further comprises an agent effective to neutralize any excess of said cross-linking fixative. Suitable neutralizing agents are described herein above.

### The Figures show:

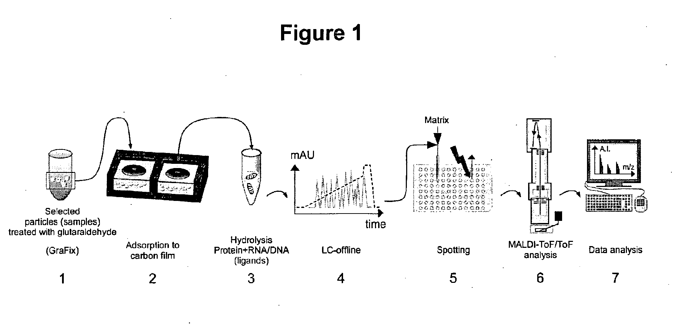

**Figure 1****:** Exemplary workflow of the method of analyzing a biological sample according to the invention. The exemplary workflow is also referred to as electron microscopy carbon-assisted digest (ECAD) of glutaraldehyde-treated protein samples.

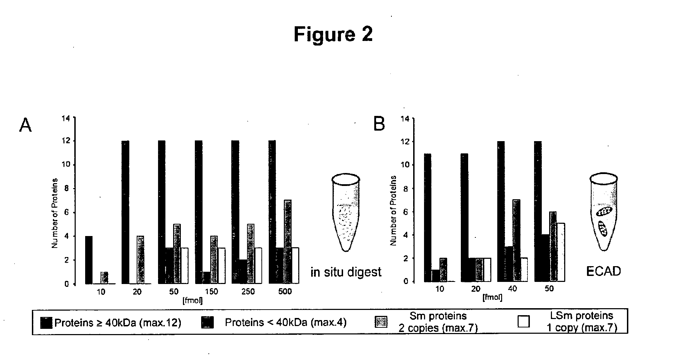

**Figure 2****:** Minimal sample amount required for *in situ* hydrolysis vs. ECAD. (A) Analysis of varying amounts of tri-snRNP particles digested in solution. In solution digest of tri-snRNPs samples was performed by denaturing samples in the presence of 4 M urea and adding ribonucleases. Samples were diluted to a urea concentration of 1 M, and the protein moiety was hydrolysed with trypsin. Peptides were further analysed by LC-MALDI-MSMS, and proteins were identified by database search. For data analysis, [U4/U6.U5] tri-snRNP-specific proteins were classified into proteins with MW ≥ or ≤ 40 kDa, Sm proteins and LSm proteins. (B) LC-MALDI-MSMS analysis of increasing amounts of tri-snRNP particles by the ECAD method. ECAD analysis was performed as in Figure 1a. The best sensitivity for MS analysis (in terms of proteins identified by LC-MALDI-MSMS and subsequent database search) was achieved at a glutaraldehyde concentration of 0.075% (v/v).

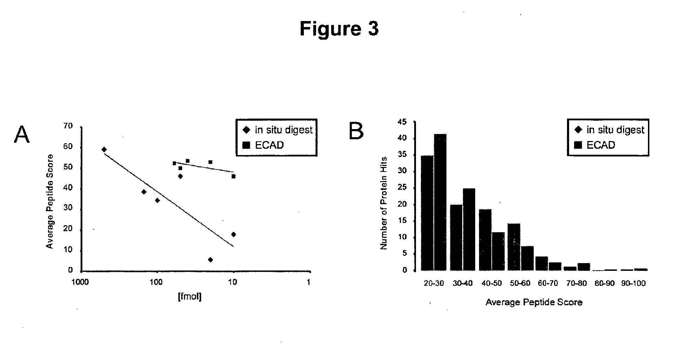

**Figure 3****:** Glutaraldehyde-treated samples show a higher degree of confidence in database search and reproducibility in protein identification. (A) The quality of the MSMS data can be described by the average peptide score (APS), describing the confidence in the database search using Mascot as search engine. Average peptide score derived from tri-snRNPs digested in solution are plotted against the quantity of sample used (three experiments) and compared with corresponding figures obtained from tri-snRNPs treated by ECAD. Significantly, MSMS spectra of peptides that identify the proteins in the database search show a significantly higher score per (non-modified) peptide than do peptides derived from the digest in solution. (B) Plot of the numbers of all proteins hits obtained in the database search against APS. In ECAD experiments a significantly greater number of proteins was identified with significant average peptide scores between 40 and 70.

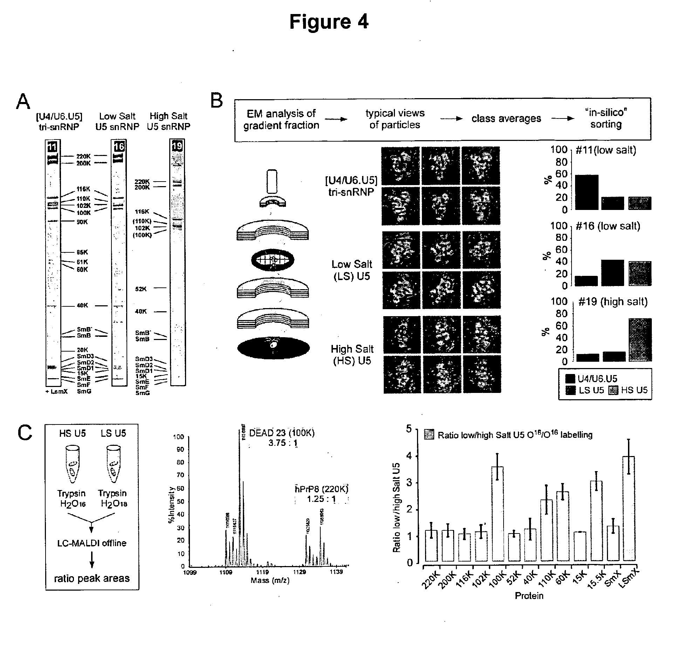

**Figure 4****:** Correlation of EM and proteome analysis in heterogeneous particle populations. Sample populations containing 25S [U4/U6.U5] tri-snRNP and low-salt and high-salt U5 snRNPs (A) were analysed by EM. *In silico* sorting of images and calculation of class averages of the structures revealed heterogeneity of the samples (B), in particular in the low-salt U5 snRNP samples. To monitor the degree of heterogeneity on the protein level, we performed the ECAD analysis of low-salt and high-salt U5 snRNPs in the presence of light and heavy water (C). As the figure shows, the quantitative proteome analysis demonstrated that the heterogeneity of the sample is due to differences in the relative abundance of the U5-specific proteins 100K and the tri-snRNP specific proteins 110K and LSm, and of the U4/U6 snRNP-specific proteins 60K and 15.5K.
**Figure 5****:** Schematics of the GraFix setup. **(a)** The GraFix gradient is prepared such that the fixation reagent is added to the denser glycerol solution. **(b)** GraFix gradients are fractionated from bottom to top, then either used directly for negative-stained electron microscopy or purified away from glycerol for unstained cryo grid preparation.

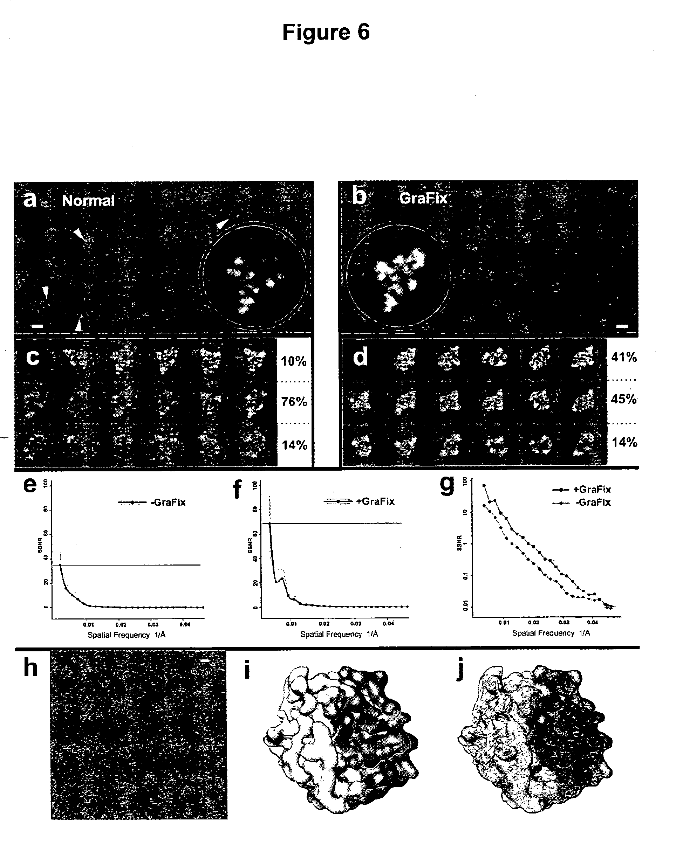

**Figure 6****:** Effect of GraFix-based sample preparation on B complex spliceosomes and the 70S ribosome. **(a,b)** Uranyl formate-stained electron microscopic raw image of spliceosomes prepared by a conventional glycerol gradient **(a)** or GraFix **(b)**. Scale bars, 40nm. Arrowheads, smaller broken parts and flexible elements. Insets, similarly oriented spliceosomal class average. **(c,d)** Class averages obtained from a set of 5,000 raw images of non-GraFix-prepared **(c)** or GraFix-prepared **(d)** samples. The average number of class members is 15 images. Class averages were sorted vertically with respect to contrast and structural definition. GraFix treatment **(d)** generates computed class averages with much improved contrast (top and middle; 86% of images), as compared to samples prepared by the conventional method **(c)**, where only ∼10% of class averages (top) show relatively well defined structural features. **(e,f)** Quantitative analysis of signal quality in class averages of non-GraFix-prepared **(e)** and GraFix-prepared **(f)** spliceosomes (gray shading, s.d.). **(g)** Logarithmic representation of data in **e** and **f**. **(h)** Unstained cryo-EM raw image of the 70S *E. coli* ribosome initiation complex after GraFix preparation and buffer exchange. **(i)** Three-dimensional reconstruction of the ribosomal 70S initiation complex at -15 A resolution. Blue, 50S subunit. Yellow, 30S subunit. Green, P-site tRNA. (j) Fit of the 70S *E. coli* X-ray structure into the electron microscopial density map.

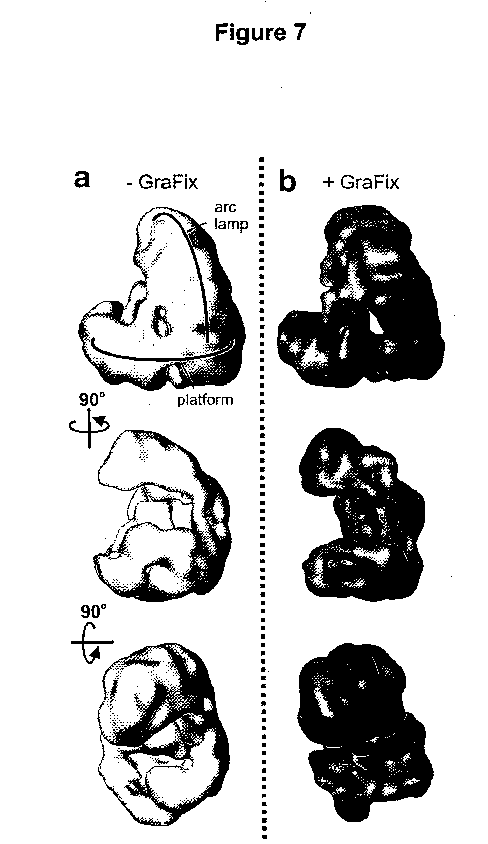

**Figure 7****:** Improved structure of GraFix-prepared APC/C. **(a,b)** The structure of non-GraFix-prepared human APC/C at a resolution of -25 A (ref. 7). **(a)** and of GraFix-prepared human APC/C at a resolution of -21 A **(b)**.

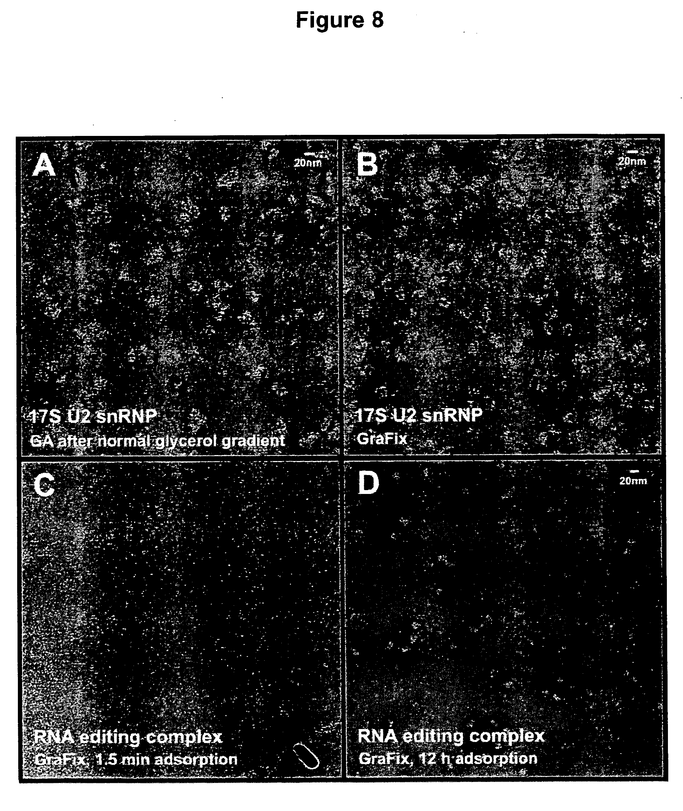

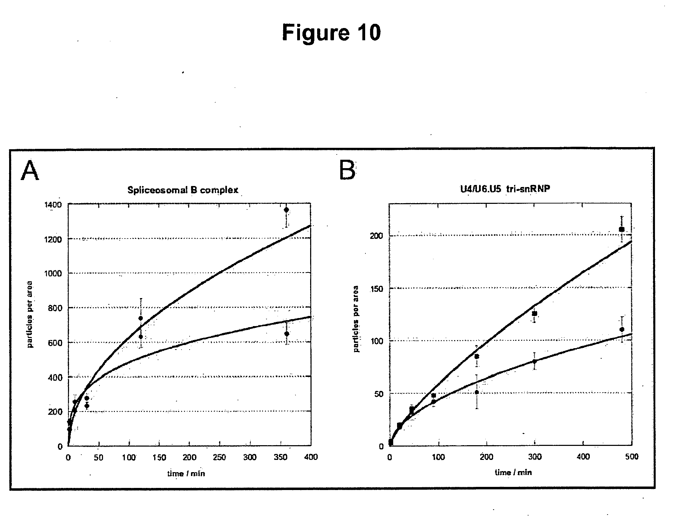

**Figure 8****:** GraFix tests on sample homogeneity, particle distribution and structural integrity. A) Negative stain image of a 17S U2 snRNP fraction of a U2 snRNP preparation, with glutaraldehyde (GA) added after standard gradient centrifugation. B) Negative stain image of 17S U2 snRNP after GraFix of the same U2 snRNP preparation reveals a considerably more homogeneous population of particles. C) No particles were detected of a GraFix treated RNA editing complex after 1.5 minutes of adsorption time. D) Extending the adsorption time (see also **Fig. 10**) of the GraFix treated *T. brucei* kinetoplastid RNA editing complex up to 12 hours leads to good quality images with sufficient particle distribution that allows further single particle analysis.

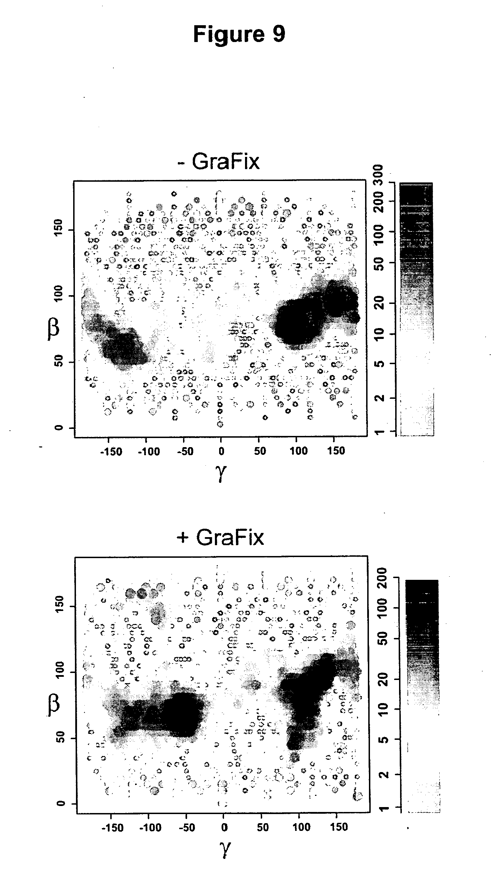

**Figure 9****:** More isotropic particle orientation of APC/C upon GraFix treatment. Projection angle distribution of the non GraFix treated APC/C reveals a highly preferential particle orientation of APC/C on the carbon support film. GraFix treatment of APC/C results in more isotropic particle orientations which is very helpful for accurate determination of projection angles and thus for reliable 3D structure determination (see also **Fig. 7**).
**Figure 10****:** Enhanced particle binding to carbon film. Particle binding to carbon film was measured for unfixed (lower graphs) and GraFix treated (upper graphs) spliceosomal B complexes (A) and U4/U6.U5 tri-snRNPs (B). While the particle concentration on carbon film shows no significant difference between unfixed and GraFix treated complexes after 2 min, roughly twice the number of GraFix particles can be bound to carbon film after ∼6 hours of adsorption avoiding the danger of particle disintegration during extended adsorption (see also **Fig. 8**).

The following examples illustrate the invention but should not be construed as being limiting.

### Example 1

### (EM) Carbon-assisted digest of glutaraldehyde-treated protein samples (ECAD)

Described is an approach to determine the proteome of glutaraldehyde-fixed protein particles on carbon films by mass spectrometry (MS). The carbon films are those used in electron microscopy (EM). The quality of the MS-generated data from ECAD-treated samples is improved, resulting in better reproducibility and higher confidence in the identification of proteins in database searches as compared with non-fixed samples. The approach is highly suitable for the analysis of low sample amounts, and it should also prove useful for the analysis of more complex samples, of membrane proteins and of other protein-ligand complexes. Also envisaged is MALDI-MS analysis of ECAD-treated samples directly from EM carbon films.

### Introduction

Mass spectrometry is the state-of-the-art technology to identify proteins. A prerequisite for the successful MS analysis (using a state-of-the-art mass spectrometer) is the choice of an appropriate method of sample preparation, in particular when one is dealing with highly complex samples, with very small amounts of sample, or with low-abundance components in the presences of other highly abundant components, highly modified and/or extremely hydrophobic or hydrophilic protein components.

In principle, protein samples are treated in two different ways before an MS-based proteome analysis is conducted: (i) Digestion *in situ* of the protein sample with endoproteinases followed by one- or multidimensional liquid chromatography (LC) of the peptides generated and tandem MSMS analysis of the peptides, or alternatively (ii) proteins are first separated by 1 D or 2D (SDS) gel electrophoresis, whereafter distinct protein bands or entire gel regions are digested with endoproteinases, and the peptides generated are extracted and separated by LC gel. Peptides are sequenced by tandem MSMS. In most cases, proteins are identified by comparing the fragment spectra of the peptides against the fragment spectra of all endoproteolytic peptides in the database, generated *in silico,* using a search engine. The peptide ion score obtained from the database search is considered to be a direct measure of confidence in the identification of the corresponding protein.

Many proteome studies face the problem of confidence in data acquisition (in particular with very small amounts of sample) and reproducibility of the analysis.

The approach according to the invention uses sample adsorption onto a solid support for digestion *in situ* of protein complexes/samples. According to this example it comprises: (i) Treatment of the sample (e.g. purified protein complex) with glutaraldehyde (between 0.025 and 0.075 % v/v); (ii) adsorption of the sample on EM carbon films; (iii) hydrolysis of proteins with endoproteinases under semi-denaturing conditions; (iv) extraction of peptides and subsequent LC-MALDI-ToF/ToF analysis. This workflow is referred to as "ECAD" (EM carbon-assisted digest).

Figure 1 shows the work flow of the ECAD approach. The approach is adapted from an embodiment of the GraFix method according to the invention, in which protein (and/or protein-ligand) complexes are separated by glycerol-gradient centrifugation in the presence of glutaraldehyde before electron microscopy is conducted. It is noted that biological samples can be treated with cross-linking fixative subsequent to or independent from gradient centrifugation.

In initial proteomic experiments with isolated protein complexes (25S [U4/U6.U5] tri-snRNPs) we reproducibly observed more peptides with a higher peptide-ion score derived from ECAD samples than in samples from *in situ* digests under semi-denaturing conditions without the ECAD procedure - as used routinely in proteomics laboratories. We envisage that the approach will be highly useful for the MS-based proteome analysis of small sample amounts (and even of entire cell lysates).

### Materials and Methods

Samples. Experiments were carried out with 25S [U4/U6.U5] tri-snRNP particles that had been purified by glycerol-gradient centrifugation. The concentration of the particles was 0.15 µg/µl.

Glutaraldehyde treatment and adsorption on EM carbon films. Particles were incubated with 0.025 - 0.075% (v/v) glutaraldehyde for 18 h. Carbon film (10 nm thick) were generated on mica by using a carbon vacuum evaporator system (Boc Edwards, Kirchheim, Germany). Coated mica was cut into pieces of approx. 2 x 4 mm. Treated samples were incubated with coated mica for 15-45 sec. Particle density on carbon films was determined by EM and revealed approximately 170 particles per 0.5 µm² (as determined by EM), corresponding to 5 fmol of particles per EM carbon film of area 8 mm².

Endoproteinase treatment. For digestion *in situ,* purified 25S [U4/U6.U5] tri-snRNPs (see above) are adjusted to a final concentration of 5 fmol/µl in 50 mM Tris.HCI at pH 7.5 containing 4 M urea (buffer A). RNA is hydrolysed for 2 h at 52 °C by adding 0.1 µg RNase A and T1 after dilution of the sample to a final urea concentration of 1 M. Samples are chilled briefly, and the proteins are digested with 1 µg trypsin (Promega) at 37 °C overnight. Digestion is stopped by adding trifluoroacetic acid (TFA) to a final concentration of 0.25% (v/v).
Samples on carbon film are treated in the following way: 5 fmol of particles bound to EM carbon film are transferred into buffer A containing 100 mM glycine to stop the reaction. Samples are sonicated for 15 min at 0 °C. The urea concentration is adjusted to 1 M, and hydrolysis of RNA and proteins are performed as above. The sample is centrifuged and the supernatant is collected. Peptides on carbon films are extracted with 60% (v/v) acetonitrile containing 0.2% (v/v) formic acid. Supernatant and extracted peptides are pooled, dried in a SpeedVac and dissolved in an appropriate volume of water with 0.025% (v/v) TFA.

MALDI-ToF/ToF analysis. Dissolved samples are injected on a Dual gradient LC-System (LC Packings) equipped with pre-columns (25 x 0.75 mm packed in-house, C18, 5 µm, 300A (Vydac, Hesperia, USA)) working in back-flush mode. Peptides are separated with a standard gradient using water, 0.1% (v/v) TFA as solvent A and 80% acetonitrile (v/v; ACN) in water, 0.1 % (v/v) TFA at a flow rate of 300 nL/min on an in-house-packed analytical column (200 x 0.75 mm, C18, 5 µm, 300A (Vydac, Hesperia, USA)). The eluate is mixed with 10 mg/mL α-cyano-4-hydroxycinnamic acid containing 10 fmol/µl Glu-Fibronigen (Sigma) as internal standard in 70% (v/v) ACN, 0.1% (v/v) TFA and spotted (Probot, LC Packings) every 15 sec onto an ABI stainless-steel target. MALDI-ToF/ToF analysis was carried out in a 4800 Analyzer (Applied Biosystems/MDS Sciex) in positive mode. For MS experiments, 800 laser shots are accumulated, and for MSMS max. 2000 laser shots are accumulated for each precursor. Acceleration voltage was 1 kV and collision energy was set to 1 x 10⁻⁶ torr. Proteins were identified by searching fragment spectra against the NCBInr database using Mascot as search engine. Two missed cleavages and oxidation and carbamylation as variable modifications were allowed for in the search. Mass deviation was 100 ppm for MS and 400 mmu for MSMS.

### Results

We first tested for sensitivity of the ECAD method. We compared various amounts of 25S [U4/U6.U5] tri-snRNP digested *in situ* with corresponding ECAD-treated samples. Figure 2 showed that 50 fmol of fixed sample on carbon support is sufficient to identify the proteins by LC-MALDI-ToF/ToF after hydrolysis of the sample on the support.

We next investigated the quality of the MS and MSMS data obtained. We compared the average peptide-ion score of samples digested *in situ* with that of ECAD-treated samples. Figure 3 shows that the fragmentation data derived from ECAD gave a significantly higher score (and thus a higher quality) as compared with non-fixed samples. ECAD will therefore prove useful for the analysis of small amounts of sample.

We applied our approach to the analysis of heterogeneous sample preparations in order to directly compare and correlate the degree of heterogeneity obtained in EM studies with that obtained in proteome analysis.

Here, we investigated high-salt- and low-salt snRNP preparations (Figure 4A); these differ in their protein composition. EM studies have shown that the fractions containing low-salt U5 snRNPs also contain 25S [U4/U6.U5] tri-snRNPs and particle that resemble high-salt U5 snRNPs. Consequently, class-average images of these fractions differ from those containing mainly tri-snRNP and high-salt U5 snRNP (Figure 4B). We applied ECAD to high-salt U5 snRNP in comparison with low-salt U5 snRNPs in the presence of light and heavy water, respectively (Figure 4C, left panel). MALDI-ToF/ToF analysis revealed the degree of heterogeneity through the higher abundance of the U5-specific proteins 100K and the tri-snRNP-specific proteins 110K and LSm, and the U4/U6 snRNP-specific proteins 60K and 15.5K in the low-salt U5 snRNP sample. Thus, ECAD yields an exact molecular representation of the species analysed by EM.

### Conclusions

By using our novel procedure ECAD, we have been able to show a direct correlation between the results of EM structure analysis, using image processing, and the results of MS-based proteomic analysis. Combining these results yields an exact molecular representation. ECAD-based quantification experiments reveal the presence of protein components that might cause sample heterogeneity. Importantly, the reproducibility and quality of the MSMS data at low sample concentrations is significantly higher for ECAD than for in-solution digests. These results demonstrate that mild glutaraldehyde treatment and adsorption of samples onto carbon (films) before protein hydrolysis is highly suitable for the analysis of low sample amounts; this is probably due to increased homogeneity of the sample upon glutaraldehyde treatment. The approach also should prove useful for the analysis of more complex samples, membrane proteins and other protein-ligand complexes. MALDI-MS analysis of ECAD-treated samples directly on EM carbon films is also envisaged.

### Example 2

### GraFix: Gradient centrifugation in combination with Fixation

The GraFix procedure combines rate zonal ultracentrifugation purification with a mild, but slowly increasing exposure of macromolecules to a cross-linking reagent under high pressure **(****Fig. 5****)**. There are several reasons for combining gradient centrifugation and chemical fixation instead of solely adding the fixation reagent to the purified sample. It has been shown that high-pressure gradient centrifugation using pressure cells can disrupt ribosomes into its subunits ^{1,2}. There is also pressure acting on the macromolecular complexes as a result of the centrifugal force in normal glycerol gradient centrifugation which may apparently be sufficient to disrupt weak aggregations while intra-molecular interactions are still preserved. This minimizes the risk of chemically fixing aggregates and inter-particle cross-linking. Inter-particle cross-linking was indeed never observed with GraFix in the typical concentration range (particles and fixation reagent) used (see **Methods** below). Second, standard purification of macromolecular complexes is very often performed in buffers containing primary amines like Tris buffer, which is incompatible with aldehyde cross-linking. Likewise, peptides used to elute a complex by immuno-affinity chromatography may also contain primary amines in addition to their N-terminus. Changing the buffer system during purification of macromolecular complexes is not trivial and often reduces quality and quantity of the purified material. To avoid these problems, a buffer exchange step can be performed during GraFix gradient centrifugation. For buffer exchange during the initial period of ultracentrifugation, a cushion containing a buffer that is compatible with chemical fixation reagents (such as HEPES in ∼5% glycerol) is placed on top of the gradient before sample loading. The cushion thus effectively avoids any initial contact of the particles and the primary amine containing buffer to the fixation reagent at beginning of centrifugation (Fig. 5).

Centrifugation conditions are chosen such that the complex sediments to about 2/3 the gradient length, corresponding to 0.07 to 0.15 % final concentration of the cross-linking reagent. The gradient is fractionated from bottom to top (Fig. 5) to avoid low molecular weight material from the top of the gradient, e.g. detergents and free peptides, to contaminate the particle fractions during fractionation.

In addition to fixation, the GraFix procedure itself represents an efficient purification step. The GraFix fractions can be used directly for negative stain and cryo negative stain preparations and even unstained cryo-EM samples can be prepared after buffer exchange (e.g. using a spin column protocol) to remove glycerol. The technique constitutes a generally applicable protocol that can be performed directly after purification by standard techniques used to isolate macromolecular complexes. We have also successfully applied GraFix to complexes in the presence of the detergent Dodecyl-Maltoside during the GraFix procedure (data not shown). Dilution as a result of the gradient centrifugation does not limit the procedure since it is possible to compensate the dilution effect by allowing GraFix fractions to adsorb to the carbon support layer for several hours, leading to a steady increase in molecules bound to the carbon film **(****Fig. 10****)**. In addition, after fixation image contrast is enhanced and at the same time reduced staining artifacts are observed in negative stain and cryo-negative stain preparations **(****Fig. 6****,** **Fig. 8****)**.

### GraFix preserves structural integrity

The GraFix procedure allows structure determination of fragile macromolecular complexes by preserving their structural integrity. As glutaraldehyde fixation has the reputation of introducing artefacts in other applications we investigated the effect of GraFix on the well-known structure of the 70S *E. coli* ribosome³. Approximately 15,000 images of a 70S initiation complex (70S·fmet-tRNA^{fMet} )⁴ were recorded after GraFix treatment, glycerol removal and cryo preparation. The 70S density map at -15A resolution is essentially identical to structures that have been determined without glutaraldehyde treatment. At the current level of resolution, no significant structural differences can be detected when compared to the 70S *E. coli* X-ray structure³ **(****Fig. 6h****-j)**. Potential artefacts that are believed to be introduced by glutaraldehyde fixation, thus clearly do not play a role for initial 3D structure determination at an intermediate resolution level. In contrast, artefacts due to disruption of non-stabilized macromolecules have more dramatic consequences for digital image processing, and these can be effectively prevented by the GraFix protocol.

### Methods

Gradients are prepared according to standard protocols. Briefly, filtered solutions of buffers containing gradient solutions (glycerol, sucrose or other carbohydrates at low and high concentrations) are prepared in tubes. 0.1-0.2% glutaraldehyde (EM grade 25%, Science Services GmbH, Munich, Germany) is added to the high density solution only. We routinely run 4 ml gradients in TH-660 (Kendro Laboratory Products GmbH, Hanau, Germany) swing-out rotors (equivalent to Beckman SW60 rotors) as small fraction volumes are sufficient for EM specimen preparation. Depending on the size of the complex, centrifugation times from 2 to 18 hours and speeds of 20,000-60,000 rpm have been used. Gradients are prepared using a gradient former (Gradient Master 107, BioComp Instruments, Canada) that first tilts the gradient tubes and then rotates them axially for a specified time⁵. Various gradient buffers can be used but the system must be free of compounds reactive with the cross-linker, e.g of primary amino groups (like Tris buffer) in case of glutaraldehyde. If the sample buffer contains such ingredients, an additional layer with a compatible low density buffer (e.g. 5-7% in case of a 4 ml 10 to 30 % glycerol gradient, 200µl) can be applied on the gradient **(****Fig. 5****)**. The freshly prepared gradient is then kept at 4°C for one hour before sample loading. Subsequently, samples are added on top of the gradient. Typically, the amount of sample added on a standard 4 ml GraFix gradient is -2-60 pmol of complexes in a maximum volume of 400 µl (typically 200 µl). Speed and time settings of the centrifuge are such that the macromolecules move -2/3 down the centrifugation tube. Usually, gradients are run with and without fixation reagent in parallel to assess whether or not the presence of the chemical fixation reagent causes any differences in particle sedimentation. Furthermore, the unfixed control gradient can be used to characterize the sample by SDS PAGE. After centrifugation, the gradient is fractionated at 4°C **(****Fig. 5****)**. In our standard setup, a capillary is used to pump out the gradient from bottom to top. Alternatively, the Brandel Isco tube piercer (Isco Inc., Lincoln, USA) can be used. The optical density can be measured during fractionation into tubes using 5 drops per fraction (∼150 µl). If required, remaining glutaraldehyde can be inactivated after fractionation by adding glycine to a final concentration of 80mM.

Negative stain and cryo-negative stain grids were prepared using glycerol containing fractions, essentially as described previously⁶. For the preparation of cryo-EM grids, glycerol is removed from the GraFix fraction applying a buffer exchange spin column (Zeba spin columns, Pierce, Rockford, IL, USA). After buffer exchange, glycerol could no longer be detected when measuring the refraction index. Cryo-grid specimen preparation is performed by filling the sample into holes drilled into a black plastic (Polyximethylen) block. Small pieces of mica covered with indirectly evaporated thin carbon film (BOC EDWARDS GmbH, Germany) are used and slowly placed into the solution under an angle, such that the carbon film floats off the mica. The carbon film can be picked up after the desired adsorption time using a holey carbon grid⁶. Prior to vitrification by plunge freezing the grid into liquid ethane, a few µl of sample are added to facilitate blotting of excess solution. Grids are stored in liquid nitrogen until they are used for imaging in the cryo electron microscope.

Cryo-EM images of the 70S ribosome were recorded on a 4kx4k CCD Camera (2x binning, TVIPS GmbH, Gauting, Germany) in a Philips CM200 FEG (FEI Company, Eindhoven, The Netherlands) using a Gatan cryo holder (Gatan GmbH, München, Germany) at 157,000x magnification. The 3D reconstruction was initially determined by angular reconstitution⁷ using a low pass filtered version of a vacant ribosome complex as a start model. The final 3D structure was obtained by projection matching using multi-reference alignment by resampling to polar coordinates⁸. APC/C were prepared +/- GraFix for cryo-negative stain and were imaged as described⁹. Identical particle statistics was used (∼16,000 images) to compare the projection angle distribution for both data sets **(****Fig. 9****)**. The final 3D structures were computed upon multi-reference alignment, classification^{10,11} and angular reconstitution⁷ of the class averages as well as directly upon projection matching. Quality improvement of the +GraFix 3D map compared to -GraFix was similar irrespective of the particular refinement scheme applied.

For computation of 2D class average SSNR, the two spliceosomal data sets comprising each -4600 images were classified into 200 classes^{10,11}. A constant number of ten images per class was used for computation of SSNR as described¹², and the mean SSNR was plotted.

### Further References

### References cited in Example 2

1. A. A. Infante and R. Baierlein, Proc Natl Acad Sci U S A 68 (8), 1780 (1971).
2. A. A. Infante, B. Demple, and J. B. Chaires, J Biol Chem 257 (1), 80 (1982).
3. V. Berk, W. Zhang, R. D. Pai et al., Proc Natl Acad Sci U S A 103 (43), 15830 (2006).
4. A. L. Konevega, N. Fischer, Y. P. Semenkov et al., Nat Struct Mol Biol 14 (4), 318 (2007).
5. D. H. Coombs and N. R. Watts, Anal Biochem 148 (1), 254 (1985).
6. M. M. Golas, B. Sander, C. L. Will et al., Science 300 (5621), 980 (2003).
7. M. van Heel, Ultramicroscopy 21, 95 (1987).
8. B. Sander, M. M. Golas, and H. Stark, J Struct Biol 143 (3), 219 (2003).
9. P. Dube, F. Herzog, C. Gieffers et al., Mol Cell 20 (6), 867 (2005).
10. M. van Heel, Ultramicroscopy 13 (1-2), 165 (1984).
11. M. van Heel and J. Frank, Ultramicroscopy 6 (2), 187 (1981).
12. M. Unser, B. L. Trus, and A. C. Steven, Ultramicroscopy 23 (1), 39 (1987).

## Claims

1. A method of analyzing a biological sample, said sample comprising one or more (poly)peptides, said method comprising
(i) bringing said biological sample into contact with a cross-linking fixative;
(ii) neutralizing any excess of said cross-linking fixative;
(iii) adsorbing the sample fixated in (i) on the surface of a solid support;
(iv) adding one or more endoproteases; and
(v) detecting of one or more cleavage products of said endoprotease(s) by mass spectrometry.

2. A method of processing a biological sample, said sample comprising one or more biological macromolecules, said method comprising
(a) exposing the sample to elevated hydrostatic pressure and, either concomitantly and/or subsequently, to a cross-linking fixative.

3. The method of claim 2, wherein step (a) is effected by centrifuging said biological sample in a gradient of density and in the presence of a cross-linking fixative.

4. The method of claim 3, wherein said cross-linking fixative is provided in a concentration gradient, and wherein the directions of said gradient of density and of said concentration gradient are the same.

5. The method of claim 3 or 4, wherein said gradient of density is a gradient of an agent, said agent being selected from the group consisting of monosaccharides, oligosaccharides, polysaccharides, alcohols with two or more -OH groups, caesium salts and iodinated compounds.

6. The method of any one of claims 3 to 5, wherein said centrifuging is performed at about 50 000 to about 1 000 000 x g for about 15 min to about 25 hours.

7. The method of any one of claims 3 to 6, further comprising
(b) harvesting at least one fraction of the gradient after centrifuging according to (a).

8. The method of claim 7, further comprising
(c) adsorbing said at least one fraction on the surface of a solid support.

9. The method of claim 2, wherein said elevated hydrostatic pressure in step (a) is created by a pressure cell.

10. The method of claim 9, wherein said elevated hydrostatic pressure is between about 5 and about 100 MPa.

11. The method of claim 2, 9 or 10, further comprising, after said exposing according to (a),
(b') neutralizing any excess of said cross-linking fixative; and
(c') adsorbing said sample on the surface of a solid support.

12. The method of any one of claims 7 to 11, further comprising
(d) adding one or more endoproteases; and
(e) detecting of one or more cleavage products of said endoprotease(s) by mass spectrometry.

13. The method of claim 1 or 12, wherein digestion by said endoprotease(s) is carried out in the presence of a denaturing agent.

14. The method of any one of claims 2 or 7 to 11, further comprising
(d') subsequently performing structural analysis of said sample or of material adsorbed on said support, respectively.

15. The method of claim 14, wherein said structural analysis is selected from electron microscopy, X-ray and light scattering and atomic force microscopy.

16. The method of any one of claims 2 to 15, wherein said biological sample comprises a complex of biological macromolecules.

17. The method of any one of the preceding claims, wherein said cross-linking fixative is a bi- or multifunctional reagent, wherein the functional groups of said reagent are capable of reacting with functional groups present on said biological macromolecules to form covalent bonds.

18. The method of any one of the preceding claims, wherein said cross-linking fixative is selected from the group consisting of glutaraldehyde, formaldehyde, Bis(Sulfosuccinimidyl) suberate, Dimethyl adipimidate, Dimethyl pimelimidate, Dimethyl Suberimidate, Dithiobis(succinimidyl) propionate, Disuccinimidyl suberate, β-[Tris(hydroxymethyl) phosphino] propionic acid, Carbodiimide, Galactose dialdehyde, S-Acetylthioacetic acid-N-hydroxysuccinimide ester, 3-Maleimidobenzoic acid *N*-hydroxysuccinimide ester, 1,4-Bis[3-(2-pyridyldithio)propionamido]butane, Bis[2-(N-succinimidyl-oxycarbonyloxy)ethyl] sulfone, Dimethyl 3,3'-dithiopropionimidate dihydrochloride, Sebacic acid bis(N-succinimidyl) ester, Tris-(2-carboxyethyl)phosphine hydrochloride, 1-Ethyl-3-(3-dimethylaminopropyl) carbodiimide hydrochloride, N-Hydroxysulfosuccinimide sodium salt, Succinimidyl acetylthioacetate, Succinimidyl trans-4-(maleimidylmethyl) cyclohexane-1-carboxylate, Succinimidyl 3-(2-pyridyldithio)propionate and mixtures thereof.

19. The method of any one of the preceding claims, wherein said cross-linking fixative is present in a concentration between about 0.001 and about 1 vol-%.

20. The method of any one of claims 1, 8 or 11 to 19, wherein said support is selected from carbon support; carbon-coated polyvinyl formal; reversed phase material; nitrocellulose; PVDF; polystyrol; silicate-based solid supports; metals; ceramics; carbides; nitrides; plastics; carbohydrate-based supports; and ion exchange resins.

21. The method of claim 20, wherein said carbon support is selected from carbon films and activated carbon.

22. The method of any one of claims 1, 8 or 11 to 21, wherein said adsorbing is allowed to occur for a period of time of at least 5 sec.

23. A kit comprising
(a) a cross-linking fixative selected from glutaraldehyde, formaldehyde, Bis(Sulfosuccinimidyl) suberate, Dimethyl adipimidate, Dimethyl pimelimidate, Dimethyl Suberimidate, Dithiobis(succinimidyl) propionate, Disuccinimidyl suberate, β-[Tris(hydroxymethyl) phosphino] propionic acid, Carbodiimide, Galactose dialdehyde, S-Acetylthioacetic acid-N-hydroxysuccinimide ester, 3-Maleimidobenzoic acid *N-*hydroxysuccinimide ester, 1,4-Bis[3-(2-pyridyldithio)propionamido]butane, Bis[2-(N-succinimidyl-oxycarbonyloxy)ethyl] sulfone, Dimethyl 3,3'-dithiopropionimidate dihydrochloride, Sebacic acid bis(N-succinimidyl) ester, Tris-(2-carboxyethyl)phosphine hydrochloride, 1-Ethyl-3-(3-dimethylaminopropyl) carbodiimide hydrochloride, *N-*Hydroxysulfosuccinimide sodium salt, Succinimidyl acetylthioacetate, Succinimidyl *trans*-4-(maleimidylmethyl) cyclohexane-1-carboxylate, Succinimidyl 3-(2-pyridyldithio)propionate and mixtures thereof;
(b) a solid support; and
(c) an endoprotease.

24. The kit of claim 23, wherein said support is selected from carbon support, nitrocellulose, PVDF, polystyrol and silicate-based solid supports.

25. The kit of claim 23 or 24, further comprising a denaturing agent.

26. The method of claim 13 or the kit of claim 25, wherein said denaturing agent is selected from urea, thiourea, guanidinium hydrochloride, guanidinium thiocyanate, acetonitril, methanol, octylglycoside, sodium dodecyl sulfate (SDS) and lithium dodecyl sulfate (LDS).

27. The method of claim 1, 12, 13 or 16 to 22 or the kit of any one of claims 23 to 26, wherein said endoprotease is selected from Trypsin, Chymotrypsin, Lys-C, Glu-C/Staphylococcus V8, Asp-N, Arg-C, Proteinase K, Pepsin, Papain, Thermolysin, Elastase and Thrombin.

28. The method of claim 13 or 16 to 22 or the kit of claim 25 or 26, wherein said endoprotease is an endoprotease which is capable of cleaving its substrate in the presence of said denaturing agent.

29. The kit of any one of claims 23 to 28, further comprising a pressure cell.

30. The kit of any one of claims 23 to 29, further comprising an agent effective to neutralize any excess of said cross-linking fixative.
